# DEMANDE DE BREVET EUROPEEN

(11) **EP 1 623 736 A1**
(43) Date de publication de la demande: **08.02.2006**
(21) Numéro de dépôt: 04103631.0
(22) Date de dépôt: 28.07.2004
(51) Int. Cl.: A61N 1/05

(54) **Pôle de stimulation auxiliaire pour sonde de stimulation cardiaque et sonde ainsi équipée**

(71) Demandeur: Bemurat, Marc, F-33850 Leognan (FR); Bemurat, Diane, F-33850 Leognan (FR); Bemurat, Laurent, F-33850 Leognan (FR); Bemurat, Eliane, F-33850 Leognan (FR)
(72) Inventeur: Bemurat, Marc, F-33850 Leognan (FR); Bemurat, Diane, F-33850 Leognan (FR); Bemurat, Laurent, F-33850 Leognan (FR); Bemurat, Eliane, F-33850 Leognan (FR)
(74) Mandataire: Thébault, Jean-Louis

(57) **Abrégé**

- L'objet de l'invention est un pôle de stimulation auxiliaire pour sonde de stimulateur cardiaque, comprenant une dérivation (3) constituée d'un élément conducteur enroulé hélicoïdalement, mono ou multifil, ladite dérivation étant, à une extrémité, connectée au conducteur ou à l'un des conducteurs de la sonde de stimulation et, à l'autre extrémité, munie d'un embout de raccordement éventuel à une source de stimulation auxiliaire, caractérisé en ce que ledit conducteur de dérivation (3) est relié au conducteur (1) ou à l'un des conducteurs de la sonde par l'intermédiaire d'un manchon (2), l'extrémité dudit conducteur de dérivation (3) étant enroulée sur au moins une fraction de tour du manchon (2) et soudée à ce dernier, la zone de jonction des conducteurs (1, 3) étant encapsulée dans un enrobage (6) d'un matériau isolant et en ce que ledit embout est constitué d'un manchon en matériau isolant enveloppant l'extrémité du conducteur de dérivation (3) et au moins en partie inséré dans un guide tubulaire en matériau isolant à l'intérieur duquel est disposé un dispositif obturateur transperçable tout en conservant son étanchéité.

## Description

La présente invention se rapporte aux sondes de stimulation de stimulateur cardiaque.

Actuellement, sur une sonde de stimulation, seule la tête de la sonde peut recevoir une stimulation électrique. Cette tête de sonde étant introduite dans le boîtier de stimulateur cardiaque n'est accessible que lorsque la sonde est déconnectée et sortie du boîtier.

Lors du remplacement d'un boîtier de stimulateur cardiaque, la sonde de stimulation est pratiquement toujours conservée. La manoeuvre consiste à déconnecter !a sonde du boîtier, à la sortir de celui-ci, à la réintroduire dans le nouveau boîtier et enfin, à reénfouir sous la peau l'ensemble sonde et boîtier.

Cette manipulation peut prendre plusieurs dizaines de secondes.

Or, dès que la sonde est déconnectée, le patient n'est plus stimulé. Cela n'a pas d'inconvénient si le patient garde un rythme cardiaque propre, mais certains patients « dépendants » n'ont aucun rythme spontané. Ils sont donc en arrêt cardiaque jusqu'au moment où la sonde de stimulation est connectée au nouveau boîtier.

Chez un patient dépendant, cette manoeuvre est délicate, voire dangereuse.

Chez ces patients totalement dépendants, il est souvent conseillé de mettre en place une sonde intra cardiaque de stimulation temporaire, connectée à un stimulateur externe, pour stimuler le coeur pendant le remplacement du boîtier.

Le pôle de stimulation accessoire permet de se connecter au stimulateur externe avant de procéder au remplacement du boîtier.

Il procure le même avantage que la sonde de stimulation temporaire. Il permet donc une stimulation permanente pendant toutes les manoeuvres nécessaires au remplacement du boîtier.

Par le brevet FR 2 654 939 déposé au nom du Demandeur, il est proposé un pôle de stimulation auxiliaire pour sonde unipolaire consistant en une dérivation formée par un conducteur spiralé connecté au conducteur de la sonde et dont l'autre extrémité est soudée à une fiche de connexion.

Le brevet FR 2 684 881 également déposé au nom du Demandeur décrit un type de dérivation analogue appliqué à la fois à une sonde unipolaire et à une sonde bipolaire à conducteurs spiralés coaxiaux.

L'invention vise à simplifier et faciliter l'accès au pôle auxiliaire tout en conservant une isolation électrique et une étanchéité optimales.

A cet effet, l'invention a pour objet un pôle de stimulation auxiliaire pour sonde de stimulateur cardiaque, comprenant une dérivation constituée d'un élément enroulé hélicoïdalement, mono ou multifil, électriquement conducteur, ladite dérivation étant, à une extrémité, connectée électriquement au conducteur ou à l'un des conducteurs de la sonde de stimulation et, à l'autre extrémité, munie d'un embout de raccordement éventuel à une source de stimulation auxiliaire, caractérisé en ce que ledit conducteur de dérivation est relié au conducteur ou à l'un des conducteurs de la sonde par l'intermédiaire d'un manchon en matériau conducteur enveloppant ledit conducteur de sonde et soudé à ce dernier, l'extrémité dudit conducteur de dérivation étant enroulée sur au moins une fraction de tour du manchon et soudé à ce dernier, la zone de jonction des conducteurs étant encapsulée dans un enrobage d'un matériau isolant électriquement et en ce que ledit embout est constitué d'un manchon en matériau électriquement isolant enveloppant l'extrémité du conducteur de dérivation et au moins en partie inséré dans un guide tubulaire en matériau électriquement isolant à l'intérieur duquel est disposé un dispositif obturateur en silicone ou analogue transperçable tout en conservant son étanchéité.

Suivant un mode de réalisation préféré, ledit pôle de stimulation auxiliaire ledit guide tubulaire comporte, disposés coaxialement audit conducteur de dérivation et dans son prolongement, deux logements de réception d'obturateurs en silicone ou analogue, séparés par un conduit axial, de diamètre réduit, de guidage d'un mandrin ou analogue de liaison électrique dudit conducteur à ladite source de stimulation auxiliaire, la mise en contact avec le conducteur s'effectuant par insertion du mandrin à l'intérieur du conducteur.

L'invention s'applique à des sondes de type unipolaire. Dans une telle application, ladite extrémité du conducteur de dérivation enroulée sur ledit manchon est enroulée en hélice sur au moins un tour complet et le conducteur de dérivation forme avec le conducteur unique de la sonde une jonction en Y dont l'angle est de l'ordre de 15 à 30°, ladite jonction étant encapsulée de manière étanche dans une enveloppe en silicone ou analogue.

L'invention s'applique également à des sondes de type bipolaire à deux conducteurs coaxiaux. Dans cette application, ladite extrémité du conducteur de dérivation est enroulée sur un manchon soudé au conducteur intérieur relié à l'électrode distale de la sonde, le conducteur extérieur étant, à hauteur de la dérivation, constitué par un second manchon électriquement conducteur, connecté au conducteur extérieur, entourant à distance le conducteur intérieur et muni d'une fenêtre pour le passage du conducteur de dérivation, un matériau isolant du type silicone ou analogue étant interposé entre, d'une part, les conducteurs interne et de dérivation et, d'autre part, ledit second manchon, cependant que la jonction ainsi formée est encapsulée dans une enveloppe étanche de silicone ou analogue.

L'invention s'applique également à des adaptateurs destinés à être connectés à des sondes unipolaires conventionnelles.

Les pôles de stimulation auxiliaire selon l'invention sont remarquables non seulement par les très bonnes qualités de contact électrique, d'isolation électrique et d'étanchéité au droit de la jonction entre le conducteur de dérivation et le conducteur de sonde mais également par la qualité et la fiabilité du contact électrique obtenu entre le conducteur de dérivation et le mandrin ou analogue relié à la source de stimulation auxiliaire. Ce contact est en effet obtenu par insertion dudit mandrin dans l'extrémité du conducteur de dérivation, à l'intérieur de ce dernier et sur une distance garantissant l'efficacité et la pérennité du contact, la structure de l'embout de raccordement garantissant par ailleurs un maintien parfait et étanche dudit mandrin.

D'autres caractéristiques et avantages du dispositif de l'invention ressortiront de la description qui va suivre de modes de réalisation, description donnée à titre d'exemple uniquement et en regard des dessins annexés sur lesquels :
- figure 1 est une vue en coupe partielle de la zone de jonction d'une dérivation de pôle de stimulation auxiliaire selon l'invention appliquée à une sonde de type unipolaire ;
- figure 2 est une vue en coupe de l'extrémité de raccordement à une source de stimulation auxiliaire de la dérivation de la figure 1 ;
- figure 3 est une vue en coupe longitudinale d'un manchon d'enveloppement de la jonction de la figure 1 ;
- figure 4 est une vue de gauche du manchon de la figure 3 ;
- figure 5 est un schéma illustrant la réalisation de la zone de jonction d'une dérivation selon l'invention appliquée à une sonde de type bipolaire ;
- figure 6 illustre la réalisation d'une dérivation sur le conducteur extérieur d'une sonde bipolaire à conducteurs coaxiaux ;
- figure 7 illustre la réalisation d'une dérivation sur l'un des conducteurs d'une sonde bipolaire à conducteurs adjacents, et
- figure 8 représente une sonde unipolaire munie d'un pôle de stimulation auxiliaire selon l'invention et connectée à un boîtier de stimulateur cardiaque.

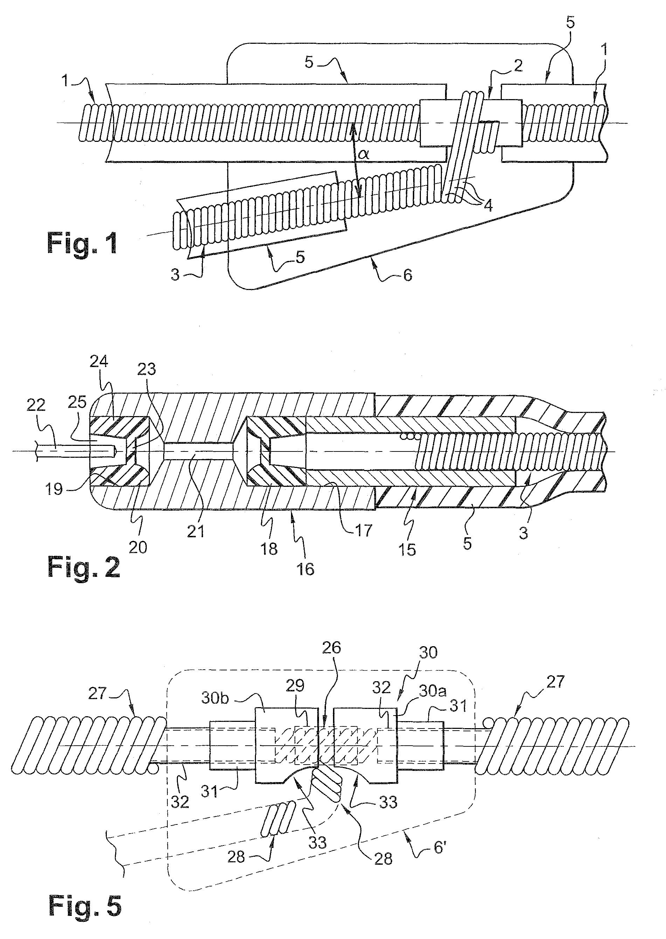

Sur la figure 1 on a représenté en 1 un conducteur filaire enroulé en hélice d'une sonde de stimulation cardiaque du type unipolaire.

En un endroit de la sonde proche de la fiche (non représentée) de connexion à un boîtier de stimulateur cardiaque, est soudé extérieurement au conducteur 1 un manchon 2 en matériau électriquement conducteur.

En 3 est représenté un conducteur filaire enroulé en hélice, identique au conducteur 1, par exemple constitué de trois fils 4 enroulés simultanément hélicoïdalement à spires jointives. Bien entendu, au lieu de conducteurs à trois brins, on pourrait utiliser des conducteurs mono-fil ou multi-fils à quatre brins par exemple.

Les trois brins du conducteur 3 sont, à l'extrémité de jonction au conducteur 1, sont déroulés et enroulés par exemple sur un tour complet autour du manchon 2 sur lequel ils sont soudés. Les soudures entre les conducteurs 1, 3 et le manchon 2 sont par exemple faites au laser.

Le conducteur de dérivation 3 est disposé de façon à faire avec le conducteur 1 une angulation α de l'ordre de 15 à 30°, le conducteur 3 étant, de préférence, tourné vers la fiche de connexion de la sonde audit boîtier.

En 5 est représentée l'enveloppe tubulaire en silicone ou analogue entourant les conducteurs 1 et 3, cette enveloppe étant interrompue dans la zone de jonction pour établir la connexion électrique. Pour assurer la continuité de l'isolation électrique les zones dénudées du conducteur 3 et du manchon 2 sont enrobées dans un matériau siliconé symbolisé en 6.

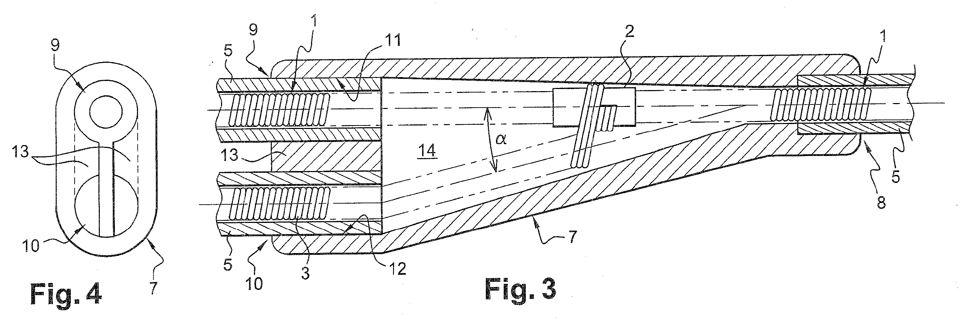

Les figures 3 et 4 illustrent un mode de réalisation d'une encapsulation de la jonction de la figure 1 à l'aide d'un manchon 7 en silicone ou analogue de forme générale triangulaire, réalisé par moulage.

Le manchon 7 comporte, à une extrémité, une sortie unique 8 tournée vers l'extrémité distale de la sonde et recevant le conducteur 1.

A l'autre extrémité, le manchon comporte deux sorties 9 et 10 adjacentes, en parallèle, recevant, l'une (9) le conducteur 1 et sa gaine 5 et, l'autre (10), le conducteur de dérivation 3 et sa gaine 5, les extrémités des gaines 5 étant reçues dans deux logements cylindriques parallèles 11 et 12 séparés par des lèvres 13.

Une injection de silicone est faite à l'intérieur du manchon 7 pour remplir l'espace interne 14 et enrober totalement les parties dénudées des conducteurs 1, 3 ainsi que le manchon 2.

Sur la figure 2, on a représenté l'autre extrémité du conducteur 3, laquelle est insérée à force au moins partiellement dans un manchon 1 5 en matériau isolant électrique, lui-même emmanché partiellement dans un second manchon 16 en matériau isolant électrique.

Le manchon 16 comporte, à une extrémité, un logement cylindrique 17 en partie occupé par le manchon 15, le reste du logement 17 étant occupé par un obturateur 18 en silicone ou analogue. L'autre extrémité du manchon 17 définit un logement cylindrique 1 9 recevant un obturateur 20 en silicone ou analogue identique à l'obturateur 18.

Entre les deux logements 17, 19 est ménagé coaxialement à ces derniers, un conduit 21 de diamètre réduit.

Les obturateurs 18, 20 ont une forme qui facilite l'insertion d'un mandrin 22 de connexion électrique relié à une source (non représentée) de stimulation auxiliaire, dans l'embout 16, puis à l'intérieur du conducteur 3.

L'obturateur d'entrée 20 présente un voile mince central 23 entouré d'une partie annulaire 24 plus massive définissant une cavité tronconique 25 de guidage du mandrin 22 en direction du voile central 23 de diamètre légèrement supérieur à celui du canal de guidage 21. Le voile 23 peut être pré-percé ou pré-fendu.

Ainsi, la pénétration du mandrin 22 dans l'embout de raccordement 16 est parfaitement guidée et l'extrémité du mandrin est parfaitement centrée sur l'axe du conducteur 3.

Le mandrin 22 est engagé à l'intérieur du conducteur 3 sur une certaine distance et parfaitement maintenu immobile par rapport à l'embout par la pression de la matière des deux obturateurs 18, 20 traversés. On a ainsi l'assurance d'avoir un contact physique entre le mandrin et le conducteur fiable et efficace électriquement parlant, tout en ayant au niveau de l'embout 1 6 une étanchéité optimale grâce au double obturateur (18, 20).

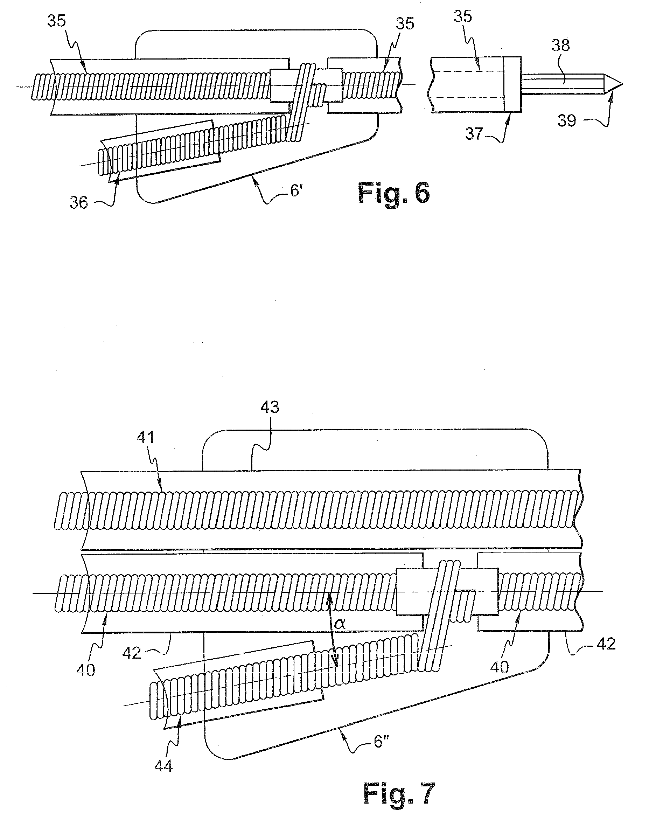

Sur la figure 5, on a illustré une application de l'invention à une sonde bipolaire à conducteurs, du type par exemple de celui de la figure 1, disposés coaxialement.

En 26 est représenté le conducteur interne, relié à l'électrode distale de la sonde (non représentée), en 27 le conducteur externe, relié à l'électrode proximale et en 28 est représenté le conducteur de dérivation électriquement connecté au conducteur interne 26.

La connexion électrique est réalisée de la même manière que sur la figure 1, à l'aide d'un manchon, symbolisé en 29 sur la figure 5, soudé autour du conducteur 26 et sur lequel extrémité des brins du conducteur 28 est enroulée sur un tour et soudée audit manchon 29.

La zone de la jonction entre conducteurs est entourée, à distance, par un manchon 30 en matériau conducteur de l'électricité connecté de part et d'autre au conducteur externe 27 qui est, bien entendu, interrompu au droit de la jonction pour effectuer la connexion avec le conducteur de dérivation 28.

Pour des questions de réalisation le manchon 30 est conformé en deux parties symétriques 30a et 30b qui seront aboutées et soudées une fois réalisée la soudure du conducteur 28 sur le manchon 29.

Chaque demi-manchon 30a, 30b est prolongé par une partie tubulaire 31 de guidage sur laquelle extrémité respective du conducteur 27 sera enfilée et soudée.

En 32 est représentée la gaine isolante entourant le conducteur interne 26 et interposée entre ce dernier et les deux manchons 30a, 30b. Cette gaine 32 est bien entendu interrompue à hauteur du manchon 29.

Le manchon externe 30 est muni d'une fenêtre 33 permettant le passage du conducteur 28 et de dimension appropriée pour que le bord de ladite fenêtre soit à une distance du conducteur 28 suffisante pour une bonne isolation électrique.

Au voisinage du manchon 29, le conducteur 28 est perpendiculaire et après la traversée de la fenêtre 33, il fait un coude dont l'angulation par rapport aux conducteurs 26, 27 est analogue à celle (α) du mode de réalisation de la figure 1.

L'espace intérieur du manchon 30, entre ce dernier et l'ensemble manchon 29 - conducteur 26, est rempli de silicone ou analogue, à des fins d'isolation électrique.

Enfin, comme dans le dispositif de la figure 1, la zone de la jonction est encapsulée dans un matériau isolant électrique 6', par exemple par mise en place d'un manchon isolant analogue au manchon 7 de la figure 3.

Sur la figure 6, on a représenté une sonde bipolaire à deux conducteurs coaxiaux , le conducteur externe 35 étant relié au conducteur de dérivation 36 de la même manière que la connexion électrique illustrée par la figure 1, la zone de jonction étant encapsulée dans un matériau siliconé 6'.

Le conducteur externe 35 est connecté à l'électrode proximale 37 en forme de bague de la sonde, cependant que le conducteur interne 38 est connecté à l'électrode distale 39. Ce mode d'application de la stimulation auxiliaire par le pôle proximal, lequel sert de simple pôle positif en stimulation normale, est néanmoins tout à fait efficace, même s'il nécessite un peu plus de puissance électrique.

Ce pôle proximal 37 ainsi utilisé en stimulation auxiliaire unipolaire constitue le pôle négatif de la stimulation.

Par ailleurs, en vue d'une telle application, il peut être intéressant de réduire sur la sonde bipolaire la distance entre le pôle distal et le pôle proximal, par exemple en ramenant l'écartement entre les parties électriquement actives des deux pôles à une dizaine de millimètres, ce qui permettra d'avoir un meilleur contact entre l'endocarde et le pôle proximal.

Sur la figure 7, on a représenté l'application de l'invention à une sonde bipolaire à deux conducteurs 40, 41 adjacents, disposés chacun dans leur gaine 42, 43.

L'un (40) des conducteurs est connecté à un conducteur de dérivation 44 de la même manière que sur la figure 1, cependant que l'autre conducteur 41 conserve son intégrité et côtoie simplement la jonction entre les conducteurs 40 et 44, et que la zone de jonction est encapsulée d'un enrobage siliconé 6" enveloppant également le conducteur 41.

Le conducteur de dérivation 44 est, de préférence, relié au conducteur (40) connecté à l'électrode distale de la sonde.

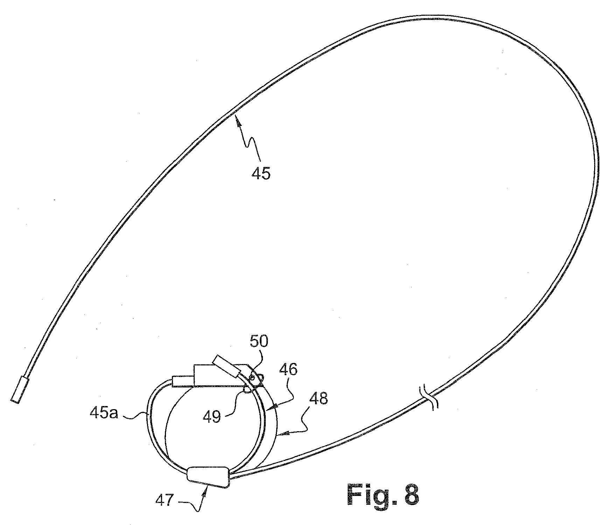

Sur la figure 8 on a représenté en 45 une sonde de type unipolaire munie d'un pôle de stimulation auxiliaire 46 selon l'invention.

La jonction de dérivation 47 est proche du boîtier 48 du stimulateur cardiaque auquel est connectée la sonde.

La longueur de la dérivation formée par le pôle auxiliaire 46 est légèrement supérieure à celle du tronçon de sonde 45a reliant la jonction 47 à la fiche de connexion au boîtier 48.

Le pôle auxiliaire 46 peut ainsi être partiellement enroulé pour épouser une partie du contour du boîtier , ce qui facilite la mise en place du stimulateur et l'extrémité du pôle 46 peut être fixée au boîtier 48 à l'aide d'un lien 49 facilement secable engagé par exemple dans un passage 50 ménagé dans le boîtier à proximité de la connexion avec la sonde.

Le trou 50 réalisé par le constructeur du boîtier est initialement destiné à la fixation du boîtier dans la poche intertissulaire d'insertion de ce dernier.

Un tel lien 49 maintient en permanence, lorsque le stimulateur est en place, l'extrémité dudit pôle 46 et la rend facilement accessible lorsqu'il sera nécessaire d'intervenir pour effectuer une stimulation auxiliaire lors du remplacement du boîtier 48.

Il est à noter que le dispositif illustré en figure 1 peut constituer un adaptateur connectable à une sonde unipolaire conventionnelle dont l'extrémité, côté stimulateur, sera sectionnée et raccordée électriquement au tronçon du conducteur 1 de droite du dispositif de la figure 1, le tronçon de conducteur 1 de gauche étant relié à une fiche conventionnelle de connexion au boîtier de stimulateur.

L'angulation formée par le conducteur de dérivation par rapport au conducteur sur lequel il est connecté peut bien entendu varier et être quelconque, y compris à 90°.

## Revendications

1. Pôle de stimulation auxiliaire pour sonde de stimulateur cardiaque, comprenant une dérivation (3) constituée d'un élément enroulé hélicoïdalement, mono ou multifil, électriquement conducteur, ladite dérivation étant, à une extrémité, connectée électriquement au conducteur ou à l'un des conducteurs de la sonde de stimulation et, à l'autre extrémité, munie d'un embout (16) de raccordement éventuel à une source de stimulation auxiliaire, **caractérisé en ce que** ledit conducteur de dérivation (3) est relié au conducteur (1) ou à l'un (26) des conducteurs de la sonde par l'intermédiaire d'un manchon (2, 29) en matériau conducteur enveloppant ledit conducteur de sonde (1, 26) et soudé à ce dernier, l'extrémité dudit conducteur de dérivation (3) étant enroulée sur au moins une fraction de tour du manchon (2, 29) et soudé à ce dernier, la zone de jonction des conducteurs (1, 3 ; 26, 28) étant encapsulée dans un enrobage (6, 6') d'un matériau isolant électriquement et **en ce que** ledit embout est constitué d'un manchon (15) en matériau électriquement isolant enveloppant l'extrémité du conducteur de dérivation (3) et au moins en partie inséré dans un guide tubulaire (16) en matériau électriquement isolant à l'intérieur duquel est disposé un dispositif obturateur (18, 20) en silicone ou analogue transperçable tout en conservant son étanchéité.

2. Pôle de stimulation auxiliaire selon la revendication 1, **caractérisé en ce que** ledit guide tubulaire (16) comporte, disposés coaxialement audit conducteur de dérivation (3) et dans son prolongement, deux logements (17,19) de réception d'obturateurs (18, 20) en silicone ou analogue, séparés par un conduit axial (21), de diamètre réduit, de guidage d'un mandrin (22) ou analogue de liaison électrique dudit conducteur (3) à ladite source de stimulation auxiliaire, la mise en contact avec le conducteur (3) s'effectuant par insertion du mandrin (22) à l'intérieur du conducteur (3).

3. Pôle de stimulation auxiliaire selon la revendication 1 ou 2, plus particulièrement destiné à une sonde de type unipolaire, **caractérisé en ce que** ladite extrémité du conducteur de dérivation (3) enroulée sur ledit manchon (2) est enroulée en hélice sur au moins un tour complet et le conducteur de dérivation (3) forme avec le conducteur unique (1) de la sonde une jonction en Y dont l'angle est de l'ordre de 15 à 30°, ladite jonction étant encapsulée de manière étanche dans une enveloppe (7) en silicone ou analogue.

4. Pôle de stimulation auxiliaire selon la revendication 1 ou 2, plus particulièrement destiné à une sonde bipolaire à deux conducteurs coaxiaux, **caractérisé en ce que** ladite extrémité du conducteur de dérivation (28) est enroulée sur un manchon (29) soudé au conducteur intérieur (26) relié à l'électrode distale de la sonde, le conducteur extérieur (27) étant, à hauteur de la dérivation, constitué par un second manchon (30) électriquement conducteur, connecté au conducteur extérieur (27), entourant à distance le conducteur intérieur (26) et muni d'une fenêtre (33) pour le passage du conducteur de dérivation (28), un matériau isolant du type silicone ou analogue étant interposé entre, d'une part, les conducteurs interne (26) et de dérivation (28) et, d'autre part, ledit second manchon (30), cependant que la jonction ainsi formée est encapsulée dans une enveloppe étanche (6') de silicone ou analogue.

5. Pôle de stimulation auxiliaire selon la revendication 1 ou 2, plus particulièrement destiné à une sonde bipolaire à deux conducteurs coaxiaux, **caractérisé en ce que** l'extrémité du conducteur de dérivation est connectée au conducteur extérieur de la sonde, la stimulation auxiliaire s'effectuant en mode unipolaire via l'électrode proximale de la sonde.

6. Pôle de stimulation auxiliaire selon la revendication 3, agencé sur un adaptateur destiné à être connecté à une sonde unipolaire.

7. Sonde de stimulation unipolaire ou bipolaire comportant un pôle de stimulation auxiliaire selon l'une des revendications 1 à 5.

8. Sonde selon la revendication 7, **caractérisé en ce que** le pôle de stimulation auxiliaire (35) présente une longueur sensiblement supérieure à celle du tronçon de sonde (34a) reliant la jonction de dérivation (36) à la fiche de connexion au boîtier du stimulateur (37), en sorte de permettre de fixer l'extrémité dudit pôle de stimulation auxiliaire (35) audit boîtier par un lien (49).
